# EUROPEAN PATENT APPLICATION

(11) **EP 3 804 641 A1**
(43) Date of publication of application: **14.04.2021**
(21) Application number: 18919554.8
(22) Date of filing: 21.09.2018
(51) Int. Cl.: A61B 17/34, A61M 25/06

(54) **DILATOR**

(30) Priority: 24.05.2018 WO PCT/JP2018/019981
(71) Applicant: ASAHI INTECC CO., LTD., Seto-shi, Aichi 489-0071 (JP)
(72) Inventor: FUSEYA, Yukihiro, Nagoya-shi, Aichi 463-0024 (JP); HOSOMI, Ryo, Nagoya-shi, Aichi 463-0024 (JP)
(74) Representative: Prinz & Partner mbB
(86) International application number: PCT/JP2018/035092
(87) International publication number: WO 2019/225026

(57) **Abstract**

A dialer capable of improving a propulsive force or reducing a resistance is provided. A dilator 1 includes a hollow shaft 7 having a tapered portion 7B with an outer diameter gradually increasing from a distal end to a proximal end, and a protruding portion 8 formed on an outer periphery of the tapered portion 7B and spirally extending along a longitudinal axis direction of the hollow shaft 7 on the outer periphery of the tapered portion 7B. The protruding portion 8 has a first protruding portion 8A located on a distal end side of the tapered portion 7B, and a second protruding portion 8B located on a proximal end side of the tapered portion 7B, and the first and second protruding portions 8A and 8B differ from each other in terms of a height from an outer peripheral surface of the tapered portion.

## Description

### TECHNICAL FIELD

The present invention relates to a dilator.

### BACKGROUND ART

For treatment, a dilator for expanding a hole formed on a wall of a lesion in a digestive tract or the like of a patient is known. The hole is expanded by inserting a distal end of the dilator into the hole formed on the wall and pressing a tapered portion into the hole. For example, such a dilator is disclosed in Patent Literature 1.

### CITATION LIST

### Patent Literature

Patent Literature 1: Japanese Unexamined Patent Application Publication No 2002-177289

### SUMMARY OF INVENTION

### Technical Problem

In the dilator, a screw thread is formed on a tapered distal end of a rod. The dilator is rotated, then the screw thread is caught by a wall of a lesion, and thereby a propulsive force is generated in the dilator. In addition, when the dilator is removed from the lesion by rotating the dilator, the screw thread receives a resistance from friction with the wall of the lesion.

Thus, an object of the present invention is to provide a dilator capable of improving a propulsive force or reducing a resistance.

### Solution to Problem

To achieve the above object, the dilator according to one aspect of the present invention includes a hollow shaft having a tapered portion with an outer diameter gradually increasing from a distal end to a proximal end, and a protruding portion formed on an outer periphery of the tapered portion and spirally extending along a longitudinal axis direction of the hollow shaft on the outer periphery of the tapered portion, in which the protruding portion has a first protruding portion located on a distal end side of the tapered portion, and a second protruding portion located on a proximal end side of the tapered portion, and the first and second protruding portions differ from each other in terms of a height from an outer peripheral surface of the tapered portion.

In addition, the height of the first protruding portion from the outer peripheral surface of the tapered portion may be smaller than the height of the second protruding portion from the outer peripheral surface of the tapered portion.

In addition, the protruding portion has a first wire wound around the outer peripheral surface of the tapered portion, and the first protruding portion has the first wire located on the distal end side of the tapered portion, and a first covering layer made of a resin for covering an outer peripheral surface of the first wire located on the distal end side of the tapered portion. The second protruding portion has the first wire located on the proximal end side of the tapered portion, and a second covering layer made of a resin for covering the outer peripheral surface of the first wire located on the proximal end side of the tapered portion, and a thickness of the first covering layer may be smaller than a thickness of the second covering layer.

In addition, the height of the second protruding portion from the outer peripheral surface of the tapered portion may be smaller than the height of the first protruding portion from the outer peripheral surface of the tapered portion.

In addition, the protruding portion has the first wire wound around the outer peripheral surface of the tapered portion, and the first protruding portion has the first wire located on a distal end portion of the tapered portion, and a first covering layer made of the resin for covering the outer peripheral surface of the first wire located on the distal end portion of the tapered portion. The second protruding portion has the first wire located on a proximal end portion of the tapered portion, and a second covering layer made of the resin for covering the outer peripheral surface of the first wire located on the proximal end portion of the tapered portion, and the thickness of the first covering layer may be larger than the thickness of the second covering layer.

### Advantageous Effects of Invention

The present invention makes it possible to provide a dilator capable of improving a propulsive force or reducing a resistance.

### BRIEF DESCRIPTION OF DRAWINGS

FIG. 1 is an overall view of a dilator according to the first embodiment.
FIG. 2 is a partial sectional view of a tapered portion in the dilator according to the first embodiment.
FIG. 3 is an overall view of a dilator according to the second embodiment.
FIG. 4 is a partial sectional view of a tapered portion in the dilator according to the second embodiment.
FIG. 5 is a partial sectional view of a tapered portion in a dilator according to the third embodiment.
FIG. 6 is a partial sectional view of a tapered portion in a dilator according to the fourth embodiment.
FIG. 7 is a partial sectional view of a tapered portion in a dilator according to a modification example.
FIG. 8 is a partial sectional view of a tapered portion in a dilator according to the fifth embodiment.
FIG. 9 is an overall view of a dilator according to the sixth embodiment.
FIG. 10 is a partial sectional view of a tapered portion of the dilator according to the sixth embodiment.
FIG. 11 is a partial sectional view of a tapered portion in a dilator according to a modification example.
FIG. 12 is a partial sectional view of a tapered portion in a dilator according to a modification example.
FIG. 13 is a partial sectional view of a tapered portion in a dilator according to a modification example.
FIG. 14 is a diagram illustrating a distal end portion in a dilator according to a modification example.
FIG. 15 is a diagram illustrating a distal end portion in a dilator according to a modification example.
FIG. 16 is a diagram illustrating a distal end portion in a dilator according to a modification example.

### DESCRIPTION OF EMBODIMENTS

Hereinafter, the embodiments of the present invention will be explained with reference to the figures. Note that sizes of the dilators illustrated in the figures are described to make it easier to understand the contents of the embodiments, and do not correspond to the actual sizes.

### <First Embodiment>

A dilator 1 according to the first embodiment of the present invention will be explained with reference to the figures.

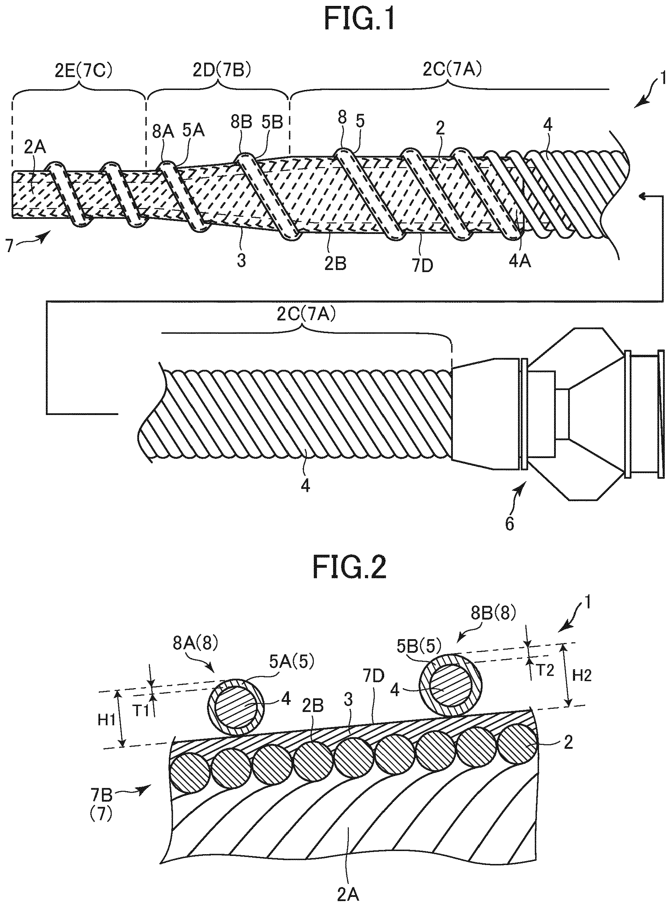

FIG. 1 is an overall view of the dilator 1 according to the first embodiment. FIG. 2 is a partial sectional view of a tapered portion 7B in the dilator 1 according to the first embodiment.

In addition, in FIG. 1 and FIG. 2, the left side of the figure refers to a distal end side (farther side) to be inserted into a body, and the right side refers to a proximal end side (hand side, nearer side) to be operated by an operator such as a surgeon.

In FIG. 1, the dilator 1 includes a first coil 2, an outer peripheral covering layer 3, a second coil 4, a coil covering layer 5, and a connector 6.

The first coil 2 is formed into a hollow shape by winding one or a plurality of metal wires. For example, the first coil 2 is formed by winding 10 metal wires made of a stainless steel. On the first coil 2, a through-hole 2A penetrating from the proximal end to the distal end is formed. The first coil 2 includes a proximal end portion 2C, a tapered portion 2D, and a distal end portion 2E.

The proximal end portion 2C is located on the proximal end side of the dilator 1, and the connector 6 is connected to the proximal end of the proximal end portion 2C. In addition, the proximal end portion 2C has a substantially constant outer diameter from the proximal end to the distal end of the proximal end portion 2C.

The tapered portion 2D is located on the distal end side of the proximal end portion 2C, and is configured to extend from the distal end of the proximal end portion 2C to the distal end side and have an outer diameter gradually decreasing toward the distal end side. In other words, the tapered portion 2D has an outer diameter gradually increasing from the distal end to the proximal end.

The distal end portion 2E is located on the distal end side of the tapered portion 2D and extends from the distal end of the tapered portion 2D to the distal end side. The distal end portion 2E has a substantially constant outer diameter from the proximal end to the distal end of the distal end portion 2E. Thus, the first coil 2 has an outer diameter gradually increasing from the distal end to the proximal end.

The outer peripheral covering layer 3 covers outer peripheral surfaces 2B of the distal end side of the proximal end portion 2C, the tapered portion 2D, and the distal end portion 2E. For example, the outer peripheral covering layer 3 is a heat-shrinkable tube. Examples of a resin constituting the outer peripheral covering layer 3 include a biocompatible resin material such as a polyamide resin and a fluororesin, a hydrophilic coating material, and the like. The outer peripheral covering layer 3 has a substantially constant thickness, but may be configured to have a thickness gradually decreasing from the proximal end side to the distal end side.

In this way, the first coil 2 and the outer peripheral covering layer 3 constitute a hollow shaft 7. In addition, the proximal end portion 2C of the first coil 2 and a part of the outer peripheral covering layer 3 covering the proximal end portion 2C constitute a proximal end portion 7A of the hollow shaft 7. The tapered portion 2D of the first coil 2 and a part of the outer peripheral covering layer 3 covering the tapered portion 2D constitute a tapered portion 7B of the hollow shaft 7. The distal end portion 2E of the first coil 2 and a part of the outer peripheral covering layer 3 covering the distal end portion 2E constitute a distal end portion 7C of the hollow shaft 7. Thus, the tapered portion 7B of the hollow shaft 7 is configured to have an outer diameter gradually decreasing from the proximal end to the distal end. In other words, the tapered portion 7B has an outer diameter gradually increasing from the distal end to the proximal end.

The second coil 4 is composed of one metal wire, in which the wire is wound around an outer peripheral surface 7D of the hollow shaft 7 in the opposite direction (Z-twisted) to the first coil 2 (S-twisted). The second coil 4 may be composed of a plurality of metal wires. The wire constituting the first coil 2 and the second coil 4 is e.g. a metal wire made of a stainless steel, a superelastic alloy such as a nickel-titanium, or the like, or a resin wire. The second coil 4 corresponds to a first wire. Herein, a pitch of the second coil 4 is not particularly limited, but the proximal end side has a densely-wound structure in which wire parts are in contact with each other, and the distal end side of the proximal end portion 7A, the tapered portion 7B, and the distal end portion 7C in the hollow shaft 7 have a coarsely-wound structure in which the wire parts are separated from each other. Thus, the second coil 4 has a gap 4A between the adjacent wire parts along a longitudinal axis direction of the hollow shaft 7.

The coil covering layer 5 covers an outer periphery of a part of the second coil 4 wound around the outer periphery of the outer peripheral covering layer 3 (a part coarsely wound in the second coil 4). The coil covering layer 5 is e.g. a heat-shrinkable tube, and a resin constituting the coil covering layer 5 is the same as the resin constituting the outer peripheral covering layer 3. The coil covering layer 5 is configured to have a thickness gradually decreasing from the proximal end side to the distal end side. Incidentally, the coil covering layer 5 may cover the second coil 4 over the whole length of the second coil 4.

Then, the part of the second coil 4 wound around the outer periphery of the outer peripheral covering layer 3 (the part coarsely wound in the second coil 4) and the coil covering layer 5 constitute a protruding portion 8 spirally extending along the longitudinal axis direction of the hollow shaft 7. Thereby, the protruding portion 8 spirally extending along the longitudinal axis direction of the hollow shaft 7 is formed on the outer periphery of the tapered portion 7B of the hollow shaft 7. The protruding portion 8 has a first protruding portion 8A located on the distal end side of the tapered portion 7B and a second protruding portion 8B located on the proximal end side of the tapered portion 7B. The screw action of the protruding portion 8 makes it possible to advance the dilator 1 also by rotating the dilator 1.

As illustrated in FIG. 2, the first protruding portion 8A is composed of the second coil 4 located on the distal end side of the tapered portion 7B, and a first covering layer 5A for covering the outer peripheral surface of the second coil 4 located on the distal end side of the tapered portion 7B in the coil covering layer 5. The second protruding portion 8B is composed of the second coil 4 located on the proximal end side of the tapered portion 7B, and a second covering layer 5B for covering the outer peripheral surface of the second coil 4 located on the proximal end side of the tapered portion 7B in the coil covering layer 5. In addition, a thickness T1 of the first covering layer 5A is structurally smaller than a thickness T2 of the second covering layer 5B. Since an outer diameter of the second coil 4 is constant over the whole length, a height H1 of the first protruding portion 8A from the outer peripheral surface 7D of the tapered portion 7B is structurally smaller than a height H2 of the second protruding portion 8B from the outer peripheral surface 7D of the tapered portion 7B. That means, the first protruding portion 8A and the second protruding portion 8B differ from each other in terms of the height from the outer peripheral surface 7D of the tapered portion 7B.

In the first embodiment and other embodiments described below, a length of the dilator is e.g. 2,000 mm, preferably 1,600 to 2,500 mm, a length of the distal end portion 2E is e.g. 10 mm, preferably 0 to 100 mm, and a length of the tapered portion 2D is e.g. 30 mm, preferably 5 to 100 mm. A distal inner diameter of the first coil 2 is e.g. 0.7 mm, preferably 0.4 to 1.0 mm, and a proximal inner diameter of the first coil 2 is e.g. 1.5 mm, preferably 1.0 to 3.0 mm. A distal outer diameter of the second coil 4 is e.g. 1.84 mm, preferably 0.8 to 3.0 mm, and a proximal outer diameter of the second coil 4 is e.g. 2.64 mm, preferably 1.4 to 5.0 mm. In addition, a diameter of the metal wire of the first coil 2 is e.g. 0.21 mm, preferably 0.1 to 0.5 mm, and a diameter of the metal wire of the second coil 4 is e.g. 0.36 mm, preferably 0.1 to 0.5 mm. A thickness of the outer peripheral covering layer 3 is e.g. 0.1 mm, preferably 0.005 to 0.300 mm, a thickness of the first covering layer 5A is e.g. 0.05 mm, preferably 0.005 to 0.300 mm, and a thickness of the second covering layer 5B is e.g. 0.1 mm, preferably 0.006 to 0.400 mm.

The connector 6 as a grip portion is a part where an operator pushes the dilator into a body and rotates the dilator. The distal end of the connector 6 is connected to the proximal end of the first coil 2 and the proximal end of the second coil 4. The connector 6 is made of a resin and has a hollow shape with a through-hole communicating with the through-hole 2A of the first coil 2.

In the dilator 1 according to the first embodiment, the first protruding portion 8A and the second protruding portion 8B differ from each other in terms of the height from the outer peripheral surface 7D of the tapered portion 7B. That means, the height H1 of the first protruding portion 8A from the outer peripheral surface 7D of the tapered portion 7B is structurally smaller than the height H2 of the second protruding portion 8B from the outer peripheral surface 7D of the tapered portion 7B. Thereby, during expansion of a lesion to be expanded by the dilator 1, a propulsive force of the dilator 1 can be improved on the proximal end side of the tapered portion 7B where tightening from the lesion is strengthened. As a result, the lesion can be easily expanded.

In addition, the thickness T1 of the first covering layer 5A constituting the first protruding portion 8A is structurally smaller than the thickness T2 of the second covering layer 5B constituting the second protruding portion 8B. Thus, it is possible to prevent from greatly changing in a bending rigidity of the dilator 1. In this way, the height of the protruding portion 8 can be easily changed to a desired height by changing the thickness of the coil covering layer 5.

Next, an example of how to use the dilator will be explained.

First, an object is punctured with an introducer needle to form a hole. Subsequently, a guide wire is inserted into a through-hole of the introducer needle, and then the introducer needle is drawn out.

Next, a proximal end of the guide wire is inserted into the through-hole of the dilator, and the dilator is inserted into the hole of the object. Subsequently, the dilator is pushed and advanced while rotating the shaft to expand the hole of the punctured part. At this time, the tapered portion advances owing to a screw action or the like of a spirally-arranged protruding portion by the rotation of the shaft, so that the hole can be smoothly expanded by the tapered portion.

### <Second Embodiment>

Next, a dilator 10 according to the second embodiment of the present invention will be explained.

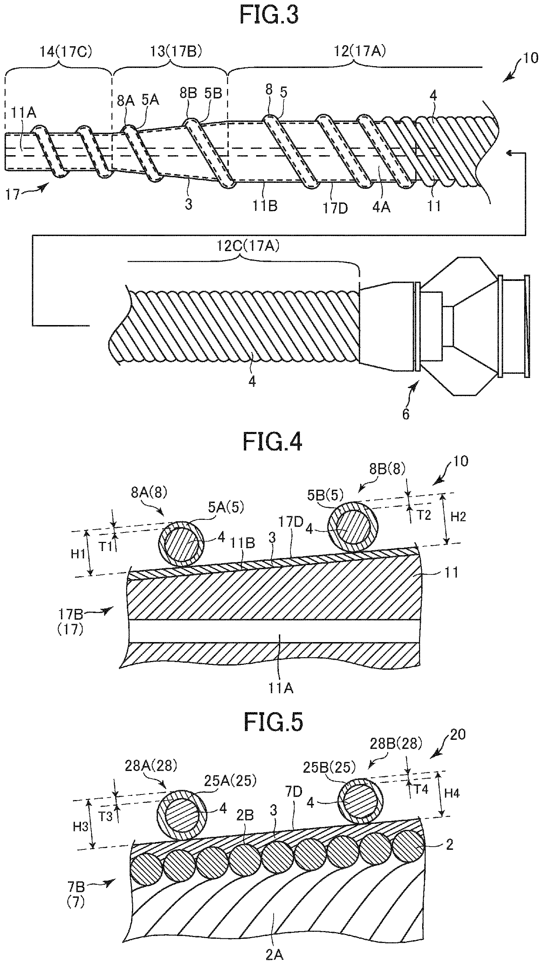

FIG. 3 is an overall view of the dilator 10 according to the second embodiment. FIG. 4 is a partial sectional view of a tapered portion 17B of the dilator 10.

In addition, in FIG. 3 and FIG. 4, the left side of the figure refers to a distal end side (farther side) to be inserted into a body, and the right side refers to a proximal end side (hand side, nearer side) to be operated by an operator such as a surgeon. Note that the same parts as those in the first embodiment are given the same symbols in the first embodiment, and explanation in the first embodiment is applied to explanation of the same parts in the second embodiment.

In FIG. 3, the dilator 10 includes a shaft main body 11, the outer peripheral covering layer 3, the second coil 4, the coil covering layer 5, and the connector 6.

The shaft main body 11 has a hollow shape having a through-hole 11A penetrating from the proximal end to the distal end. In addition, the shaft main body 11 includes a proximal end portion 12, a tapered portion 13, and a distal end portion 14.

A material constituting the shaft main body 11 is not particularly limited as long as softness of the tapered portion 13 and the distal end portion 14 is ensured and the material is biocompatible. For example, the shaft main body 11 is made of a stainless steel, a superelastic alloy material such as a nickel-titanium alloy, or a synthetic resin such as a polyvinyl chloride resin, a urethane resin, a polyolefin resin, a polyamide resin, and a fluororesin. The shaft main body 11 is formed by casting or the like.

The proximal end portion 12 is located on the proximal end side of the dilator 10, and the connector 6 is connected to the proximal end of the proximal end portion 12. In addition, the proximal end portion 12 has a substantially constant outer diameter from the proximal end to the distal end of the proximal end portion 12.

The tapered portion 13 is connected to the distal end of the proximal end portion 12, extends from the distal end of the proximal end portion 12 to the distal end side, and has a shape tapering toward the distal end side. That means, the tapered portion 13 is configured such that an outer shape on the distal end side is smaller than an outer shape on the proximal end side. In other words, the tapered portion 13 has an outer diameter gradually increasing from the distal end to the proximal end.

The distal end portion 14 is connected to the distal end of the tapered portion 13 and extends from the distal end of the tapered portion 13 to the distal end side. The distal end portion 14 has a substantially constant outer diameter from the proximal end to the distal end of the distal end portion 14. Thus, the shaft main body 11 has a hollow shape in which a distal outer diameter is smaller than a proximal outer diameter.

The outer peripheral covering layer 3 covers outer peripheral surfaces 11B of the distal end side of the proximal end portion 12, the tapered portion 13, and the distal end portion 14.

The shaft main body 11 and the outer peripheral covering layer 3 constitute a hollow shaft 17. In addition, the proximal end portion 12 of the shaft main body 11 and a part of the outer peripheral covering layer 3 covering the proximal end portion 12 constitute a proximal end portion 17A of the hollow shaft 17. The tapered portion 13 of the shaft main body 11 and a part of the outer peripheral covering layer 3 covering the tapered portion 13 constitute a tapered portion 17B of the hollow shaft 17. The distal end portion 14 of the shaft main body 11 and a part of the outer peripheral covering layer 3 covering the distal end portion 14 constitute a distal end portion 17C of the hollow shaft 17. Thus, the tapered portion 17B of the hollow shaft 17 is configured to have an outer diameter gradually decreasing from the proximal end to the distal end. In other words, the tapered portion 17B has an outer diameter gradually increasing from the distal end to the proximal end.

In addition, the second coil 4 and the coil covering layer 5 are configured in the same manner as for the dilator 1 according to the first embodiment. Thereby, the protruding portion 8 spirally extending along a longitudinal axis direction of the hollow shaft 17 is formed on an outer periphery of the tapered portion 17B of the hollow shaft 17.

As illustrated in FIG. 4, in the same manner as for the dilator 1 according to the first embodiment, the thickness T1 of the first covering layer 5A is structurally smaller than the thickness T2 of the second covering layer 5B, and the second coil 4 has the constant outer diameter over the whole length, and therefore a height H1 of the first protruding portion 8A from an outer peripheral surface 17D of the tapered portion 17B is structurally smaller than a height H2 of the second protruding portion 8B from the outer peripheral surface 17D of the tapered portion 17B. That means, the first protruding portion 8A and the second protruding portion 8B differ from each other in terms of the height from the outer peripheral surface 17D of the tapered portion 17B.

The dilator 10 according to the second embodiment also exhibits the same effect as of the dilator 1 according to the first embodiment.

### <Third Embodiment>

Next, a dilator 20 according to the third embodiment of the present invention will be explained.

FIG. 5 is a partial sectional view of a tapered portion 7B of the dilator 20 according to the third embodiment.

In addition, in FIG. 5, the left side of the figure refers to a distal end side (farther side) to be inserted into a body, and the right side refers to a proximal end side (hand side, nearer side) to be operated by an operator such as a surgeon.

The dilator 20 includes the first coil 2, the outer peripheral covering layer 3, the second coil 4, a coil covering layer 25, and the connector 6 (FIG. 1). The dilator 20 differs from the dilator according to the first embodiment in terms of the coil covering layer 25. Since the configurations of the first coil 2, the outer peripheral covering layer 3, the second coil 4, and the connector 6 are the same as in the first embodiment, explanation of their configurations in the first embodiment is applied to explanation of the configurations in the third embodiment.

In the same manner as for the coil covering layer 5 according to the first embodiment, the coil covering layer 25 covers an outer periphery of a part of the second coil 4 wound around the outer periphery of the outer peripheral covering layer 3 (a part coarsely wound in the second coil 4). A material constituting the coil covering layer 25 is the same as for the coil covering layer 5 according to the first embodiment. The coil covering layer 25 is configured to have a thickness gradually increasing from the proximal end side to the distal end side. Incidentally, the coil covering layer 25 may cover the second coil 4 over the whole length of the second coil 4.

Then, the part of the second coil 4 wound around the outer periphery of the outer peripheral covering layer 3 (the part coarsely wound in the second coil 4) and the coil covering layer 25 constitute a protruding portion 28 spirally extending along the longitudinal axis direction of the hollow shaft 7. Thereby, the protruding portion 28 spirally extending along the longitudinal axis direction of the hollow shaft 7 is formed on the outer periphery of the tapered portion 7B of the hollow shaft 7. The protruding portion 28 has a first protruding portion 28A located on the distal end side of the tapered portion 7B, and a second protruding portion 28B located on the proximal end side of the tapered portion 7B.

The first protruding portion 28A is composed of the second coil 4 located on the distal end side of the tapered portion 7B, and a first covering layer 25A for covering the outer peripheral surface of the second coil 4 located on the distal end side of the tapered portion 7B in the coil covering layer 25. The second protruding portion 28B is composed of the second coil 4 located on the proximal end side of the tapered portion 7B, and a second covering layer 25B for covering the outer peripheral surface of the second coil 4 located on the proximal end side of the tapered portion 7B in the coil covering layer 25. In addition, a thickness T3 of the first covering layer 25A is structurally larger than a thickness T4 of the second covering layer 25B. Since the outer diameter of the second coil 4 is constant over the whole length, a height H3 of the first protruding portion 28A from the outer peripheral surface 7D of the tapered portion 7B is structurally larger than a height H4 of the second protruding portion 28B from the outer peripheral surface 7D of the tapered portion 7B. That means, the first protruding portion 28A and the second protruding portion 28B differ from each other in terms of the height from the outer peripheral surface 7D of the tapered portion 7B.

In the dilator 20 according to the third embodiment, the first protruding portion 28A and the second protruding portion 28B differ from each other in terms of the height from the outer peripheral surface 7D of the tapered portion 7B. That means, the height H3 of the first protruding portion 28A from the outer peripheral surface 7D of the tapered portion 7B is structurally larger than the height H4 of the second protruding portion 28B from the outer peripheral surface 7D of the tapered portion 7B. Thereby, during removal of the dilator 20 from a lesion, a resistance from the lesion on the proximal end side of the tapered portion 7B can be reduced. As a result, the dilator 20 can be easily removed from the lesion.

In addition, the thickness T3 of the first covering layer 25A constituting the first protruding portion 28A is structurally larger than the thickness T4 of the second covering layer 25B constituting the second protruding portion 28B. Thus, it is possible to prevent from greatly changing in a bending rigidity of the dilator 20. In this way, the height of the protruding portion 28 can be easily changed to a desired height by changing the thickness of the coil covering layer 25.

In addition, the coil covering layer 25 according to the third embodiment may be applied to the coil covering layer 5 of the dilator 10 according to the second embodiment. The second coil 4 is covered with the coil covering layer 25 instead of the coil covering layer 5 of the dilator 10 according to the second embodiment, so that a dilator that exhibits the same effect as of the dilator 20 according to the third embodiment can be provided.

### <Fourth Embodiment>

Next, a dilator 30 according to the fourth embodiment of the present invention will be explained.

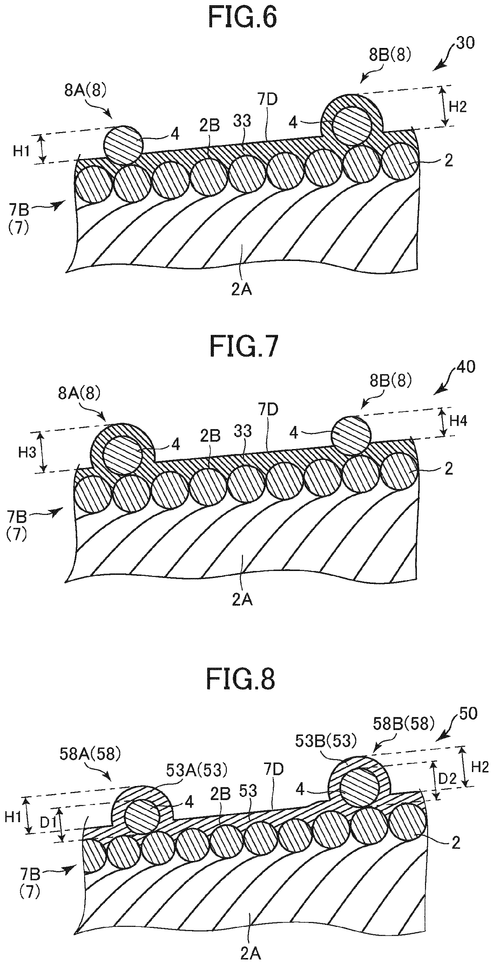

FIG. 6 is a partial sectional view of the tapered portion 7B of the dilator 30 according to the fourth embodiment.

In addition, in FIG. 6, the left side of the figure refers to a distal end side (farther side) to be inserted into a body, and the right side refers to a proximal end side (hand side, nearer side) to be operated by an operator such as a surgeon.

The dilator 30 includes the first coil 2, a covering layer 33, the second coil 4, and the connector 6 (FIG. 1). The dilator 30 differs from the dilator according to the first embodiment in that the covering layer 33 is used instead of the outer peripheral covering layer 3 and the coil covering layer 5 according to the first embodiment. Since the configurations of the first coil 2, the second coil 4, and the connector 6 are the same as in the first embodiment, explanation of their configurations in the first embodiment is applied to explanation of the configurations in the fourth embodiment.

In the dilator 30 according to the fourth embodiment, a second coil 4 is wound around the outer peripheral surfaces 2B of the distal end side of the proximal end portion 2C, the tapered portion 2D, and the distal end portion 2E in direct contact with each other. In addition, the whole outer periphery of the distal end side of the proximal end portion 2C, the tapered portion 2D, and the distal end portion 2E, and the second coil 4 formed on the outer peripheral surfaces 2B thereof is covered with the covering layer 33 having the substantially constant thickness. However, a second coil 4 located on the distal end side of the tapered portion 7B is exposed without being covered with the covering layer 33. A material constituting the covering layer 33 is the same as for the outer peripheral covering layer 3 according to the first embodiment. The covering layer 33 has a substantially constant thickness, but may be configured to have a thickness gradually decreasing from the proximal end side to the distal end side.

In the fourth embodiment, the first coil 2 and a part of the covering layer 33 covering the outer peripheral surface 2B of the first coil 2 constitute the hollow shaft 7. The second coil 4 and a part of the covering layer 33 covering the second coil 4, as well as a part of the second coil 4 exposed from the covering layer 33 constitute the protruding portion 8 spirally extending along the longitudinal axis direction of the hollow shaft 7.

As a result, the first protruding portion 8A is composed only of the second coil 4, and the second protruding portion 8B is composed of the second coil 4 and the covering layer 33. According to the configuration, the height H1 of the first protruding portion 8Afrom the outer peripheral surface 7D of the tapered portion 7B is smaller than the height H2 of the second protruding portion 8B from the outer peripheral surface 7D of the tapered portion 7B.

The dilator 30 having the aforementioned configuration makes it possible to improve a propulsive force of the dilator 30 on the proximal end side of the tapered portion 7B where tightening from a lesion is strengthened during expansion of the lesion to be expanded by the dilator 30. As a result, the lesion can be easily expanded.

In addition, as with a dilator 40 illustrated in FIG. 7, the tapered portion 7B may be configured such that the outer periphery of the second coil 4 located on the distal end side is covered with the covering layer 33, and the outer periphery of the second coil 4 located on the proximal end side is exposed.

The first protruding portion 8A is composed of the second coil 4 and the covering layer 33, and the second protruding portion 8B is composed only of the second coil 4. According to the configuration, a height H3 of the first protruding portion 8A from the outer peripheral surface 7D of the tapered portion 7B is larger than a height H4 of the second protruding portion 8B from the outer peripheral surface 7D of the tapered portion 7B.

The dilator 40 having the aforementioned configuration makes it possible to reduce a resistance from the lesion on the proximal end side of the tapered portion 7B during removal of the dilator 40 from the lesion. As a result, the dilator 40 can be easily removed from the lesion.

In addition, instead of the first coil 2, the shaft main body 11 of the dilator 10 according to the second embodiment may be applied to the dilators 30 and 40 illustrated in FIG. 6 and FIG. 7.

<Fifth Embodiment>

Next, a dilator 50 according to the fifth embodiment of the present invention will be explained.

FIG. 8 is a partial sectional view of the tapered portion 7B of the dilator 50 according to the fifth embodiment.

Additionally, in FIG. 8, the left side of the figure refers to a distal end side (farther side) to be inserted into a body, and the right side refers to a proximal end side (hand side, nearer side) to be operated by an operator such as a surgeon.

The dilator 50 includes the first coil 2, a covering layer 53, the second coil 4, and the connector 6 (FIG. 1). The dilator 50 differs from the dilator according to the first embodiment in terms of the configuration of the first coil 2, the configuration of the second coil 4, and use of the covering layer 53 instead of the outer peripheral covering layer 3 and the coil covering layer 5 according to the first embodiment. Since the configuration of the connector 6 is the same as in the first embodiment, detailed explanation of the connector 6 is omitted.

The configurations of the first coil 2 and the second coil 4 are the same as of the first coil 2 and the second coil 4 according to the first embodiment except that the wire diameter on the distal end side is smaller than the wire diameter on the proximal end side in the fifth embodiment, and therefore explanation of their configurations in the first embodiment is applied to explanation of the configurations in the fifth embodiment excluding the wire diameters of the first coil 2 and the second coil 4.

The first coil 2 is configured such that the wire diameter on the distal end side is smaller than the wire diameter on the proximal end side. Specifically, the wire diameter of the first coil 2 on the distal end side of the tapered portion 7B is structurally smaller than the wire diameter of the first coil 2 on the proximal end side of the tapered portion 7B. Similarly, the second coil 4 is also configured such that the wire diameter on the distal end side is smaller than the wire diameter on the proximal end side. Specifically, as illustrated in FIG. 8, the second coil 4 is configured such that a wire diameter D1 on the distal end side of the tapered portion 7B is smaller than a wire diameter D2 on the proximal end side of the tapered portion 7B.

The first coil 2 and the second coil 4 can be configured in such a way that the second coil having the constant wire diameter from the proximal end to the distal end is wound around the first coil having the constant wire diameter from the proximal end to the distal end in direct contact with each other, and then e.g. the distal end side of the coil is subjected to electropolishing or the like. The electropolishing is performed e.g. in such a way that a region for polishing in the coil is immersed in an electropolishing liquid, and in this state, electric power is applied to the electropolishing liquid. As a result, the surface of the coil is melted, so that the wire diameter of the coil can be decreased.

The whole outer periphery of the distal end side of the proximal end portion 2C, the tapered portion 2D, and the distal end portion 2E, and the second coil 4 formed on the outer peripheral surfaces 2B thereof is covered with the covering layer 53 having a substantially constant thickness. A material constituting the covering layer 53 is the same as for the outer peripheral covering layer 3 according to the first embodiment.

In the fifth embodiment, the first coil 2, and a part of the covering layer 53 covering the outer peripheral surface 2B of the first coil 2 constitute the hollow shaft 7. The second coil 4, and a part of the covering layer 53 covering the second coil 4 constitute a protruding portion 58 spirally extending along the longitudinal axis direction of the hollow shaft 7 on the outer periphery of the tapered portion 7B in the hollow shaft 7. Thereby a first protruding portion 58A is composed of the second coil 4 located on the distal end side of the tapered portion 7B, and a first covering layer 53A for covering the outer peripheral surface of the second coil 4 located on the distal end side of the tapered portion 7B in the covering layer 53. A second protruding portion 58B is composed of the second coil 4 located on the proximal end side of the tapered portion 7B, and a second covering layer 53B for covering the outer peripheral surface of the second coil 4 located on the proximal end side of the tapered portion 7B in the covering layer 53.

The wire diameter D1 of the second coil 4 located on the distal end side of the tapered portion 7B is structurally smaller than the wire diameter D2 of the second coil 4 located on the proximal end side of the tapered portion 7B, and the covering layer 53 has a substantially constant thickness. Thus, a height H1 of the first protruding portion 58Afrom the outer peripheral surface 7D of the tapered portion 7B is smaller than a height H2 of the second protruding portion 58B from the outer peripheral surface 7D of the tapered portion 7B. Thereby, during expansion of a lesion to be expanded by the dilator 50, a propulsive force of the dilator 50 can be improved on the proximal end side of the tapered portion 7B where tightening from the lesion is strengthened. As a result, the lesion can be easily expanded.

### <Sixth Embodiment>

Next, a dilator 60 according to the sixth embodiment of the present invention will be explained.

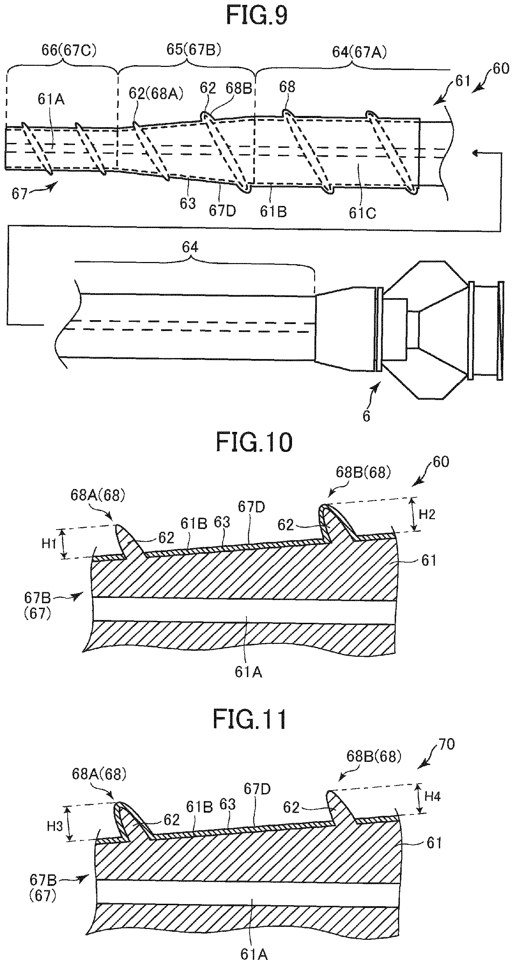

FIG. 9 is an overall view of the dilator 60 according to the sixth embodiment. FIG. 10 is a partial sectional view of a tapered portion 67B of the dilator 60.

In addition, in FIG. 9 and FIG. 10, the left side of the figure refers to a distal end side (farther side) to be inserted into a body, and the right side refers to a proximal end side (hand side, nearer side) to be operated by an operator such as a surgeon.

The dilator 60 includes a shaft main body 61, a spirally extending protruding portion 62, a covering layer 63, and the connector 6.

The shaft main body 61 has a hollow shape having a through-hole 61A penetrating from the proximal end to the distal end. A material constituting the shaft main body 61 and the protruding portion 62 is the same as the material constituting the shaft main body 11 of the dilator 10 according to the second embodiment.

In addition, the shaft main body 61 includes a proximal end portion 64, a tapered portion 65, and a distal end portion 66. In the shaft main body 61, configurations of the proximal end portion 64, the tapered portion 65, and the distal end portion 66 are the same as the configurations of the proximal end portion 12, the tapered portion 13, and the distal end portion 14 in the shaft main body 11 according to the second embodiment.

The protruding portion 62 is formed so as to project outward on an outer peripheral surface 61B of the shaft main body 61. The protruding portion 62 is spirally formed along the longitudinal axis direction on the distal end side portion of the proximal end portion 64, the tapered portion 65, and the distal end portion 66, and has a gap 61C between the adjacent protruding parts along the longitudinal axis direction of the shaft main body 61. That means, the adjacent protruding parts of the protruding portion 62 are separated from each other. The protruding portion 62 is configured integrally with the shaft main body 61 by casting or the like. In addition, the protruding portion 62 is configured to have a substantially constant height from the outer peripheral surface 61B of the shaft main body 61.

The covering layer 63 covers the outer peripheral surfaces 61B of the distal end side of the proximal end portion 64, the tapered portion 65, and the distal end portion 66. A material constituting the covering layer 63 is the same as for the outer peripheral covering layer 3 according to the first embodiment. The covering layer 63 has a substantially constant thickness, but may be configured to have a thickness gradually decreasing from the proximal end side to the distal end side.

The shaft main body 61 and the covering layer 63 constitute a hollow shaft 67. In addition, the proximal end portion 64 of the shaft main body 61 and a part of the covering layer 63 covering the proximal end portion 64 constitute a proximal end portion 67A of the hollow shaft 67. The tapered portion 65 of the shaft main body 61 and a part of the covering layer 63 covering the tapered portion 65 constitute the tapered portion 67B of the hollow shaft 67. The distal end portion 66 of the shaft main body 61 and a part of the covering layer 63 covering the distal end portion 66 constitute a distal end portion 67C of the hollow shaft 67. Thus, the tapered portion 67B of the hollow shaft 67 is configured to have an outer diameter gradually decreasing from the proximal end to the distal end. In other words, the tapered portion 67B has an outer diameter gradually increasing from the distal end to the proximal end.

In addition, the covering layer 63 covers a part of the protruding portion 62 of the proximal end side of the dilator 60 located past the proximal end side of the tapered portion 67B, but does not cover a part of the protruding portion 62 of the distal end side of the dilator 60 located past the distal end side of the tapered portion 67B . Thus, a part of the protruding portion 62 of the distal end side of the dilator 60, located past the distal end side of the tapered portion 67B, is exposed. In the sixth embodiment, the protruding portion 62 and the part of the covering layer 63 covering the protruding portion 62, as well as the part of the protruding portion 62 exposed from the covering layer 63 constitute a protruding portion 68 spirally extending along the longitudinal axis direction of the hollow shaft 67 on the outer periphery of the hollow shaft 67.

As illustrated in FIG. 10, in the protruding portion 68, a first protruding portion 68A is composed only of the protruding portion 62, and a second protruding portion 68B is composed of the protruding portion 62 and the covering layer 63. According to the configuration, a height H1 of the first protruding portion 68A from an outer peripheral surface 67D of the tapered portion 67B is smaller than a height H2 of the second protruding portion 68B from the outer peripheral surface 67D of the tapered portion 67B.

The dilator 60 having the aforementioned configuration makes it possible to improve a propulsive force of the dilator 60 on the proximal end side of the tapered portion 67B where tightening from a lesion is strengthened during expansion of the lesion to be expanded by the dilator 60. As a result, the lesion can be easily expanded.

In addition, as with a dilator 70 illustrated in FIG. 11, the tapered portion 67B may be configured such that an outer periphery of the protruding portion 62 located on the distal end side is covered with the covering layer 63, and the outer periphery of the protruding portion 62 located on the proximal end side is exposed.

The first protruding portion 68A is composed of the protruding portion 62 and the covering layer 63, and the second protruding portion 68B is composed only of the protruding portion 62. According to the configuration, a height H3 of the first protruding portion 68A from the outer peripheral surface 67D of the tapered portion 67B is larger than a height H4 of the second protruding portion 68B from the outer peripheral surface 67D of the tapered portion 67B.

The dilator 70 having the aforementioned configuration makes it possible to reduce a resistance from the lesion on the proximal end side of the tapered portion 67B during removal of the dilator 70 from the lesion. As a result, the dilator 70 can be easily removed from the lesion.

### <Seventh Embodiment>

Next, a dilator 80 according to the seventh embodiment of the present invention will be explained.

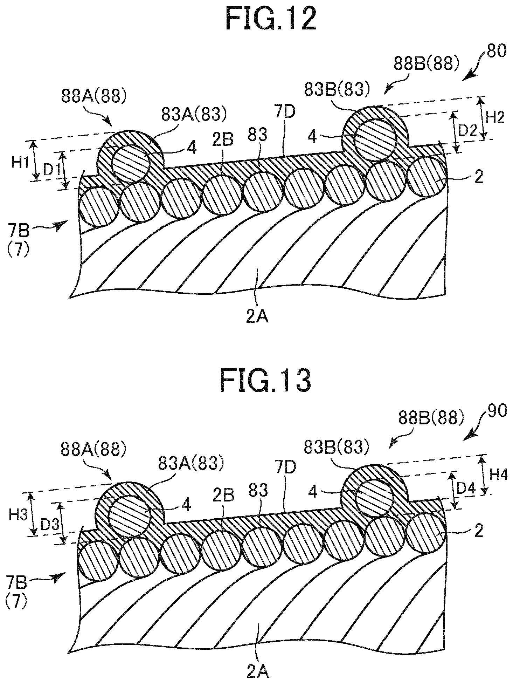

FIG. 12 is a partial sectional view of the tapered portion 7B of the dilator 80 according to the seventh embodiment.

In addition, in FIG. 12, the left side of the figure refers to a distal end side (farther side) to be inserted into a body, and the right side refers to a proximal end side (hand side, nearer side) to be operated by an operator such as a surgeon.

The dilator 80 includes the first coil 2, a covering layer 83, the second coil 4, and the connector 6 (FIG. 1). The dilator 80 differs from the dilator according to the first embodiment in terms of the configuration of the second coil 4 and use of the covering layer 83 instead of the outer peripheral covering layer 3 and the coil covering layer 5 according to the first embodiment. Since the configurations of the first coil 2 and the connector 6 are the same as in the first embodiment, detailed explanation of the first coil 2 and the connector 6 is omitted.

The configuration of the second coil 4 is the same as of the second coil 4 according to the first embodiment except that the wire diameter on the distal end side is smaller than the wire diameter on the proximal end side in the seventh embodiment, and therefore explanation of the configuration of the second coil 4 according to the first embodiment is applied to explanation of the second coil 4 according to the seventh embodiment excluding the wire diameter of the second coil 4.

The second coil 4 is configured such that the wire diameter on the distal end side is smaller than the wire diameter on the proximal end side. Specifically, as illustrated in FIG. 12, the second coil 4 is configured such that the wire diameter D1 on the distal end side of the tapered portion 7B is smaller than the wire diameter D2 on the proximal end side of the tapered portion 7B. The second coil 4 can be configured by electropolishing or the like of the distal end side of the second coil having the constant wire diameter from the proximal end to the distal end. Then, the second coil 4 subjected to electropolishing or the like is wound around the first coil 2 in direct contact with each other, to obtain the configurations of the first coil 2 and the second coil 4 illustrated in FIG. 12.

The whole outer periphery of the distal end side of the proximal end portion 2C, the tapered portion 2D, and the distal end portion 2E, and the second coil 4 formed on the outer peripheral surfaces 2B thereof is covered with the covering layer 83 having a substantially constant thickness. A material constituting the covering layer 83 is the same as for the outer peripheral covering layer 3 according to the first embodiment.

In the seventh embodiment, the first coil 2, and a part of the covering layer 83 covering the outer peripheral surface 2B of the first coil 2 constitute the hollow shaft 7. The second coil 4, and a part of the covering layer 83 covering the second coil 4 constitute a protruding portion 88 spirally extending along the longitudinal axis direction of the hollow shaft 7 on the outer periphery of the tapered portion 7B in the hollow shaft 7. Thereby a first protruding portion 88A is composed of the second coil 4 located on the distal end side of the tapered portion 7B, and a first covering layer 83A for covering the outer peripheral surface of the second coil 4 located on the distal end side of the tapered portion 7B in the covering layer 83. A second protruding portion 88B is composed of the second coil 4 located on the proximal end side of the tapered portion 7B, and a second covering layer 83B for covering the outer peripheral surface of the second coil 4 located on the proximal end side of the tapered portion 7B in the covering layer 83.

The wire diameter D1 of the second coil 4 located on the distal end side of the tapered portion 7B is structurally smaller than the wire diameter D2 of the second coil 4 located on the proximal end side of the tapered portion 7B, and the covering layer 83 has a substantially constant thickness. Thus, a height H1 of the first protruding portion 88A from the outer peripheral surface 7D of the tapered portion 7B is smaller than a height H2 of the second protruding portion 88B from the outer peripheral surface 7D of the tapered portion 7B. Thereby, during expansion of a lesion to be expanded by the dilator 80, a propulsive force of the dilator 80 can be improved on the proximal end side of the tapered portion 7B where tightening from the lesion is strengthened. As a result, the lesion can be easily expanded.

In addition, the dilator 80 in FIG. 12 may be configured such that the wire diameter on the distal end side is larger than the wire diameter on the proximal end side in the second coil 4. That means, the second coil 4 of a dilator 90 illustrated in FIG. 13 may be configured such that a wire diameter D3 on the distal end side of the tapered portion 7B is larger than a wire diameter D4 on the proximal end side of the tapered portion 7B.

The wire diameter D3 of the second coil 4 located on the distal end side of the tapered portion 7B is structurally larger than the wire diameter D4 of the second coil 4 located on the proximal end side of the tapered portion 7B, and the covering layer 83 has the substantially constant thickness. Thus, a height H3 of the first protruding portion 88A from the outer peripheral surface 7D of the tapered portion 7B is larger than a height H4 of the second protruding portion 88B from the outer peripheral surface 7D of the tapered portion 7B.

The dilator 90 having the aforementioned configuration makes it possible to reduce a resistance from a lesion on the proximal end side of the tapered portion 7B during removal of the dilator 90 from the lesion. As a result, the dilator 90 can be easily removed from the lesion.

Although the embodiments according to the present invention have been described above, the present invention is not limited to the aforementioned embodiments and can be variously modified.

For example, the dilators 1 and 10 according to the aforementioned embodiments may be dilators 1 and 10 in which the second coil 4 has a gap between the adjacent protruding parts along the longitudinal axis direction of the first coil 2 to the proximal end of the first coil 3.

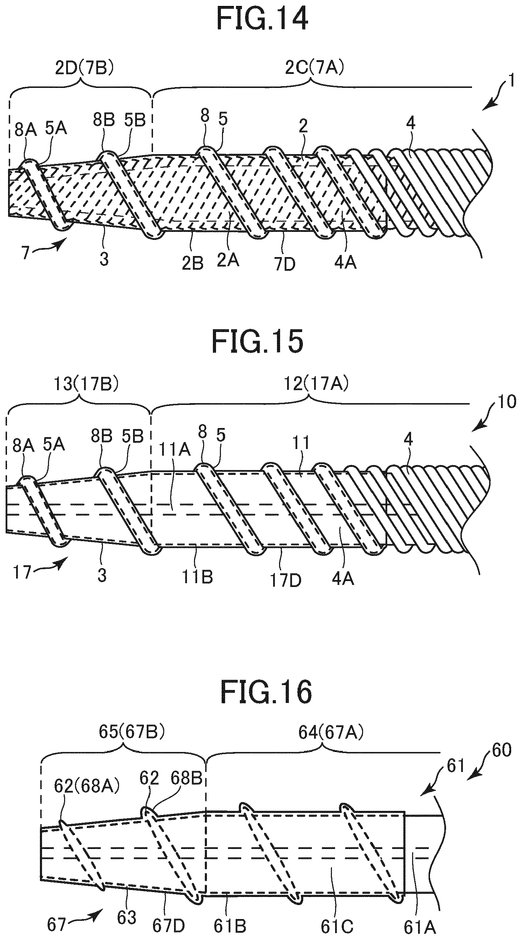

In addition, as illustrated in FIG. 14, the hollow shaft 7 of the dilator 1 according to the first embodiment need not have the distal end portion 2E. As illustrated in FIG. 15, the hollow shaft 17 of the dilator 10 according to the third embodiment need not have the distal end portion 17C. As illustrated in FIG. 16, the hollow shaft 67 of the dilator 60 according to the sixth embodiment need not have the distal end portion 67C.

In addition, the dilator 50 according to the fifth embodiment in FIG. 8 may be configured such that the shaft main body 61 of the dilator 60 according to the sixth embodiment in FIG. 10 is used instead of the first coil 2 and the second coil 4, and the distal end side of the shaft main body 61 is subjected to electropolishing or the like so that the protruding portion 62 on the distal end side of the tapered portion 65 is lower than the protruding portion 62 on the proximal end side, and thereby these protruding portions 62 are covered with the covering layer 53.

In addition, in the aforementioned embodiments, the first coil 2 has been explained as a hollow coil body composed of 10 wires, but the number of the wires is not limited to 10, and the coil may be composed of one wire or a plurality of wires.

In the embodiments illustrated in FIG. 1 to FIG. 16, it is preferable that the spirally-arranged protruding portion does not constitute a blade. The dilators according to the embodiments are intended to expand a hole previously formed on an object (e.g. a wall of a digestive tract such as a stomach of a patient). This is because, if the spirally-arranged protruding portion constitutes a blade, a living tissue on an inner face of the hole is damaged.

Consequently, it is preferable that the spirally-arranged protruding portion has a sectional shape (e.g. a sectional shape orthogonal to the spiral direction of the spirally-arranged protruding portion 62 illustrated in FIG. 10) that does not have an acute-angled corner on the radial-directional outer end portion of the shaft. That means, it is preferable that the end portion has e.g. a region composed of an obtuse-angled corner, or a shape including a curved line (e.g. a curved line containing a part of a circle or an ellipse).

### DESCRIPTION OF REFERENCE NUMERALS

- 1, 10, 20, 30, 40, 50, 60, 70, 80, 90: Dilator
- 2: First coil
- 4: Second coil
- 7, 17, 67: Hollow shaft
- 7D, 17D, 67D: Outer peripheral surface
- 8, 28, 58, 68, 88: Protruding portion
- 8A, 28A, 58A, 68A, 88A: First protruding portion
- 8B, 28B, 58B, 68B, 88B: Second protruding portion
- 5A, 25A, 53A, 83A: First covering layer
- 5B, 25B, 53B, 83B: Second covering layer

## Claims

1. A dilator comprising a hollow shaft having a tapered portion with an outer diameter gradually increasing from a distal end to a proximal end, and
a protruding portion formed on an outer periphery of the tapered portion and spirally extending along a longitudinal axis direction of the hollow shaft on the outer periphery of the tapered portion, wherein
the protruding portion has a first protruding portion located on a distal end side of the tapered portion, and a second protruding portion located on a proximal end side of the tapered portion, and
the first and second protruding portions differ from each other in terms of a height from an outer peripheral surface of the tapered portion.

2. The dilator according to claim 1, wherein
a height of the first protruding portion from the outer peripheral surface of the tapered portion is smaller than a height of the second protruding portion from the outer peripheral surface of the tapered portion.

3. The dilator according to claim 2, wherein
the protruding portion has a first wire wound around the outer peripheral surface of the tapered portion,
the first protruding portion has the first wire located on the distal end side of the tapered portion, and a first covering layer made of a resin for covering an outer peripheral surface of the first wire located on the distal end side of the tapered portion,
the second protruding portion has the first wire located on the proximal end side of the tapered portion, and a second covering layer made of a resin for covering the outer peripheral surface of the first wire located on the proximal end side of the tapered portion, and
a thickness of the first covering layer is smaller than a thickness of the second covering layer.

4. The dilator according to claim 1, wherein the height of the second protruding portion from the outer peripheral surface of the tapered portion is smaller than the height of the first protruding portion from the outer peripheral surface of the tapered portion.

5. The dilator according to claim 4, wherein
the protruding portion has the first wire wound around the outer peripheral surface of the tapered portion,
the first protruding portion has the first wire located on a distal end portion of the tapered portion, and the first covering layer made of the resin for covering the outer peripheral surface of the first wire located on the distal end portion of the tapered portion,
the second protruding portion has the first wire located on a proximal end portion of the tapered portion, and the second covering layer made of the resin for covering the outer peripheral surface of the first wire located on the proximal end portion of the tapered portion, and
the thickness of the first covering layer is larger than the thickness of the second covering layer.
